# EUROPEAN PATENT APPLICATION

(11) **EP 4 847 617 A1**
(43) Date of publication of application: **02.12.2026**
(21) Application number: 25825601.5
(22) Date of filing: 19.06.2025
(51) Int. Cl.: C12N 15/113, A61K 31/7088, A61P 35/00

(54) **USE OF NUCLEIC ACID IN PREPARATION OF DRUG FOR PREVENTING AND TREATING CACHEXIA AND ADMINISTRATION METHOD THEREFOR**

(30) Priority: 24.06.2024 CN 202410817427
(71) Applicant: Jiangsu Nutai Biologics Co., Ltd., Taizhou, Jiangsu 225300 (CN)
(72) Inventor: HU, Xiaoyun, Taizhou, Jiangsu 225300 (CN); TIAN, Chunliang, Taizhou, Jiangsu 225300 (CN)
(74) Representative: Winter, Brandl - Partnerschaft mbB
(86) International application number: PCT/CN2025/102168
(87) International publication number: WO 2026/001832

(57) **Abstract**

Use of a nucleic acid in the preparation of a drug for preventing and treating cachexia and an administration method therefor. The nucleic acid regulates in vivo cachexia-related factors by means of an oligonucleotide that the nucleic acid comprises or produces, initiates an autologous reprogramming state, regulates the activation/silencing of a key nucleic acid factor to enable a non-coding RNA regulatory signal programming network, and achieves the purpose of a single non-coding RNA regulating multiple signaling pathways, thereby enabling the establishment of new homeostasis from a dysregulated physiological state.

## Description

This invention claims the priority right to the Chinese patent application with the application number 202410817427.4, entitled Use of Nucleic Acid in Preparation of Drug for Preventing and Treating Cachexia and Administration Method Therefor, filed with the China National Intellectual Property Administration on June 24, 2024, the entire content of which is incorporated herein by reference.

### TECHNICAL FIELD

The invention belongs to the technical field of biomedicine, and particularly relates to the use of nucleic acid in preparation of drug for preventing and treating cachexia and an administration method.

### BACKGROUND ART

Cachexia is a complex metabolic clinical syndrome characterized by consumption of the body's own tissues, which can occur in various diseases such as tumors, AIDS, severe trauma, postoperative conditions, malabsorption, and sepsis. The most common type is tumor-associated cachexia, also known as cancer cachexia. Typical clinical symptoms of cachexia include progression of systemic inflammation, glycolytic metabolic mode marked by the Warburg effect due to hypoxia in the tumor microenvironment, insomnia, loss of appetite, weight loss caused by muscle wasting, systemic failure, and metabolic abnormalities.

For cancer cachexia, the General Office of the National Health Commission of the People's Republic of China issued the Guidelines for clinical diagnosis and treatment of cancer cachexia (2020 Edition), which points out that there is a lack of effective treatments for cancer cachexia and the therapeutic effects are unsatisfactory. Clinical randomized trials have shown that appetite stimulants including progestogen analogs, 5-hydroxytryptamine (5-HT) antagonists, and anti-inflammatory drugs including cyclooxygenase-2 (COX-2) inhibitors cannot completely reverse the abnormal state of cancer cachexia and are associated with risks such as thromboembolism. In addition, anti-tumor therapies such as radiotherapy and chemotherapy exacerbate muscle wasting, a major symptom of cachexia.

Muscle wasting and weight loss are important indicators for the diagnosis and treatment of cachexia. At present, reversing weight loss in patients with cancer cachexia, thereby assisting in the treatment of cancer cachexia and improving the quality of life of patients with advanced cancer, is a research hotspot in patient-centered cancer therapy.

### SUMMARY OF THE INVENTION

To solve at least some of the problems in the prior art, the invention verifies that the nucleic acid sequences of the invention can restore the normal expression of A2AR and treat cachexia. A seed sequence is creatively selected, and the designed nucleic acid sequence can act as a "sponge" to adsorb and restore the function of the A2AR gene in patients with cachexia, for use in preventing and treating cachexia. The invention designs and prepares nucleic acid sequences that can be used for preventing and treating cachexia, which include or can generate oligonucleotides *in vivo,* exist in cells and the circulatory system, restore the expression of the A2AR gene, further regulate the expression of related pathway proteins, and ultimately significantly alleviate cachexia. In the invention, *in vitro* experiments confirm that the nucleotide sequences of the invention can restore the A2AR gene, and *in vivo* experimental models verify the therapeutic effect of the oligonucleotides on cachexia. Specifically, the invention includes the following contents.

In a first aspect, the invention provides an oligonucleotide, wherein the oligonucleotide is of a following (1) and/or (2):
(1) an oligonucleotide having a sequence as shown in any one of SEQ ID NO. 1-42;
(2) an oligonucleotide obtained by nucleotide modification of the oligonucleotide in (1) above with the same function.

Specifically, the oligonucleotide has a sequence as shown in any one of SEQ ID NO. 1, 2, 4, 5, 9, 13, 14, 15, 19, 22, 23, 25, 26, 30, 34, 35, 36, or 40.

Specifically, the nucleotide modification includes modification of a phosphate group, a ribose group, and/or a conjugated linker; wherein the modification of the phosphate group refers to modification of oxygen in the phosphate group, including phosphorothioate and boranophosphate, so as to stabilize the nucleic acid structure; the modification of the ribose group refers to modification of the 2'-hydroxyl group in the ribose group, including introduction of a methoxy group or a fluorine group at the 2'-hydroxyl position of the ribose group, so that ribonucleases are less likely to cleave the nucleic acid and the stability of the nucleic acid is increased; the conjugated linker modification includes small molecule ligands, antibodies, peptides, or carbon chains.

In another aspect, the invention provides an expression vector comprising any one of the above oligonucleotides.

In certain embodiments, the expression vector is an animal cell expression vector.

In certain embodiments, the expression vector is a recombinant plasmid, an AAV viral vector, a lentiviral vector, or a retroviral vector.

In another aspect, the invention provides a use of a nucleic acid in the preparation of a drug for preventing and treating cachexia, wherein the nucleic acid comprises or can generate an oligonucleotide of the following (1) and/or (2):
(1) an oligonucleotide having a sequence as shown in any one of SEQ ID NO. 1-42;
(2) an oligonucleotide obtained by nucleotide modification of the oligonucleotide in (1) above with the same function.

In certain embodiments, according to the use of the invention, the nucleotide modification includes modification of a phosphate group and/or a ribose group and/or a conjugated linker;
wherein the modification of the phosphate group refers to any modification of the phosphate group, including phosphorothioate and boranophosphate, so as to stabilize the nucleic acid structure;
the modification of the ribose group refers to any modification of the ribose group, including introduction of a methoxy group or a fluorine group at the 2'-hydroxyl position of the ribose group, so that ribonucleases are less likely to cleave the nucleic acid and the stability of the nucleic acid is increased.

The conjugated linker modification includes small molecule ligands, antibodies, peptides, carbon chains, and other molecules.

In certain embodiments, according to the use of the invention, the nucleic acid that is able to generate the oligonucleotide of (1) and/or (2) is an animal cell expression vector.

In certain embodiments, according to the use of the invention, the vector used for the nucleic acid that is able to generate the oligonucleotide of (1) and/or (2) is a recombinant plasmid, an AAV viral vector, a lentiviral vector, or a retroviral vector.

In certain embodiments, according to the use of the invention, the oligonucleotide is contained in an extracellular vesicle.

In certain embodiments, according to the use of the invention, the dosage form of the drug includes an injection, an injectable powder, a tablet, an ointment, a capsule, a granule, an aerosol, a spray, or a powder for inhalation.

In certain embodiments, according to the use of the invention, the drug is administered systemically, including subcutaneous injection, intramuscular injection, intravenous administration, oral administration, inhalation administration, or sustained-release administration.

In certain embodiments, according to the use of the invention, the drug is designed to administer the oligonucleotide at a dose of 4 µg-60 µg per person per time based on a body weight of 60 kg.

In certain embodiments, according to the use of the invention, the drug is designed to administer the oligonucleotide at a dose of 0.005-1 µg/kg body weight.

In certain embodiments, according to the use of the invention, the drug is designed such that the effective concentration of the oligonucleotide in the systemic circulation is 1×10⁴-1×10⁹ copies/µL, or the effective concentration of the oligonucleotide in a target organ is 1×10⁴-1×10⁹ copies/mg.

In certain embodiments, according to the use of the invention, the prevention and treatment include: preventing, alleviating, reversing, improving, or curing cachexia or symptoms thereof.

In certain embodiments, according to the use of the invention, the cachexia includes one or more of the pre-cachexia stage, cachexia stage, and refractory cachexia stage caused by tumor progression.

In certain embodiments, the tumor includes a solid tumor or a non-solid tumor;
the solid tumor includes: gastric cancer, pancreatic cancer, lung cancer, intestinal cancer, prostate cancer, esophageal cancer, liver cancer, breast cancer, melanoma, osteosarcoma, neuroblastoma, rhabdomyosarcoma, Ewing's sarcoma, bladder cancer, ovarian cancer, cervical cancer, nasopharyngeal cancer, laryngeal cancer, kidney cancer, head and neck tumor, testicular cancer, thyroid cancer, or brain cancer;
the non-solid tumor includes: leukemia or lymphoma.

In certain embodiments, the tumor includes gastric cancer, lung cancer, intestinal cancer, pancreatic cancer, or liver cancer.

In certain embodiments, according to the use of the invention, the symptoms of cachexia include muscle wasting, weight loss, listlessness, insomnia, systemic failure, metabolic abnormalities, decreased diet, and/or decreased activity.

In another aspect, the invention provides a method for modulating cell proliferation *in vitro*, comprising the step of contacting an *in vitro* cell with the oligonucleotide of the invention. Preferably, the *in vitro* cell includes a tumor cell or an immortalized cell.

In another aspect, the invention provides a method for administering a nucleic acid-containing drug, wherein the nucleic acid comprises or can generate an oligonucleotide of the following (1) and/or (2):
(1) an oligonucleotide having a sequence as shown in any one of SEQ ID NO. 1-42;
(2) an oligonucleotide obtained by nucleotide modification of the oligonucleotide in (1) above with the same function;
the method comprises administering the drug systemically.

In certain embodiments, the method for administering a nucleic acid-containing drug according to the invention comprises systemic administration by subcutaneous injection, intramuscular injection, intravenous administration, oral administration, inhalation administration, or sustained-release administration.

In certain embodiments, the method for administering a nucleic acid-containing drug according to the invention is such that the drug can deliver the oligonucleotide into the body at a dose of 4 µg-60 µg per person per time based on a body weight of 60 kg.

In certain embodiments, the method for administering a nucleic acid-containing drug according to the invention is such that the drug can deliver the oligonucleotide into the body at a dose of 5 µg-50 µg per person per time based on a body weight of 60 kg.

In certain embodiments, the method for administering a nucleic acid-containing drug according to the invention is such that the drug can make the effective concentration of the oligonucleotide in the systemic circulation 1×10⁴-1×10⁹ copies/µL, or make the effective concentration of the oligonucleotide in a target organ 1×10⁴-1×10⁹ copies/mg.

The nucleic acid of the invention regulates nucleic acid factors associated with cachexia *in vivo* through the oligonucleotide contained or generated thereby, initiates autologous reprogramming, regulates the activation/silencing of key nucleic acid factors, realizes a non-coding RNA-regulated signal programming network, achieves the purpose of a single non-coding RNA regulating multiple signal pathways, and further reconstructs a new homeostasis of the disordered physiological state. Compared with other nucleic acid drugs, the drug taking the nucleic acid of the invention as an active ingredient has an extremely low dosage during prevention and treatment, ensures drug safety on the basis of achieving therapeutic effects, and subverts the current status of administration of traditional plasmid/nucleic acid drugs. In addition, the nucleic acid of the invention has no specific reaction and high *in vivo* safety.

### BRIEF DESCRIPTION OF THE DRAWINGS

FIG 1 shows the effect of an exemplary oligonucleotide expression plasmid on the expression of a target oligonucleotide in SK-Hep-1, HepG2, and H292 cell lines. (a) SEQ ID NO. 19 oligonucleotide expression plasmid in SK-Hep-1 cell line; (b) SEQ ID NO. 5 oligonucleotide expression plasmid in HepG2 cell line; (c) SEQ ID NO. 5 oligonucleotide expression plasmid in H292 cell line.
FIG 2 shows the effect of an exemplary oligonucleotide on the expression level of A2AR mRNA in different cell lines. (a) Changes in A2AR mRNA expression level over time in MKN45 cell line; (b) Times of up-regulation of A2AR mRNA in three different cell lines compared with the control group.
FIG 3 shows the effect of an exemplary oligonucleotide on the expression level of A2AR protein in CFPAC-1, CW-2, and BxPC-3 cell lines. (a) Times of up-regulation of A2AR protein in CFPAC-1 and CW-2 cell lines compared with the control group; (b) Times of up-regulation of A2AR protein in CW-2 and BxPC-3 cell lines compared with the control group; (c) Times of up-regulation of A2AR protein in BxPC-3 and SW620 cell lines compared with the control group.
FIG 4 shows the effect of a modified exemplary oligonucleotide on the expression level of A2AR protein in SW620 and CFPAC-1 cell lines.
FIG 5 shows the effect of an exemplary oligonucleotide on nude mice with lung cancer cachexia. (a) Expression level of A2AR protein in tumor tissue; (b) Body weight growth rate; (c) Food intake; (d) Water intake; (e) Tumor volume; (f) Tumor number; (g) Tumor inhibition rate.
FIG 6 shows the effect of an exemplary oligonucleotide on nude mice with lung cancer cachexia. (a) Food intake; (b) Water intake; (c) Body weight growth of the oligonucleotide group compared with the control group; (d) Tumor number; (e) Tumor volume; (f) Tumor inhibition rate.
FIG 7 shows the effect of an exemplary oligonucleotide on nude mice with pancreatic cancer cachexia. (a) Survival rate; (b) Food intake; (c) Clinical observation score; (d) Body weight growth rate.
FIG 8 shows the effect of an exemplary oligonucleotide on nude mice with intestinal cancer cachexia. (a) Body weight growth rate; (b) Tumor inhibition rate.

### DETAILED DESCRIPTION

Various exemplary embodiments of the invention will now be described in detail. Such detailed description should not be construed as a limitation of the invention, but rather as a more detailed description of certain aspects, features, and embodiments of the invention.

It should be understood that the terminology used herein is for the purpose of describing particular embodiments only and is not intended to limit the invention. In addition, for numerical ranges in the invention, it should be understood that each intermediate value between the upper and lower limits and therebetween is specifically disclosed. Every smaller range between any stated value or intermediate value within a stated range and any other stated value or intermediate value within that range is also included in the invention. The upper and lower limits of these smaller ranges may independently be included or excluded in the range.

Unless otherwise defined, all technical and scientific terms used herein have the same meaning as commonly understood by one of ordinary skill in the art to which this invention belongs. Although only preferred methods and materials of the invention are described herein, any methods and materials similar or equivalent to those described herein can be used in the practice or testing of the invention. All documents mentioned in this specification are incorporated herein by reference to disclose and describe the methods and/or materials related to the documents. In case of conflict with any incorporated document, the content of this specification shall prevail.

Experimental methods without specifying specific conditions in the specific examples are generally performed according to conventional conditions, such as those described in Sambrook et al., Molecular Cloning: A Laboratory Manual (New York: Cold Spring Harbor Laboratory Press, 1989), or according to the conditions recommended by the manufacturer.

Studies have found that cells actively secrete "nucleic acid factors" extracellularly through exosome mechanisms, globally altering the body's immunity and metabolism *in vivo.* Such actively secreted "nucleic acid factors", like physiological regulatory factors such as cytokines and chemokines, participate in *in vivo* reprogramming and systemic regulation processes. The invention administers drugs with reference to the mechanism of endogenous "nucleic acid factors", restores genes with suppressed expression in the cachexia state, resolves the imbalance of physiological state in patients with cachexia, improves cachexia symptoms, especially cancer cachexia, and enhances the quality of life of patients.

In the invention, the term "cachexia" refers to a complex metabolic clinical syndrome in which the patient's own tissues are consumed, characterized by anorexia, weight loss, systemic failure, and metabolic abnormalities. It can occur in various diseases such as cardiovascular diseases, tumors, AIDS, severe trauma, postoperative conditions, malabsorption, and sepsis. It is recognized in the art that cachexia is characterized by persistent skeletal muscle wasting, with or without adipose tissue loss, which cannot be completely alleviated by conventional nutritional therapy. At present, the only drug officially marketed for cachexia is Anamorelin produced by Ono Pharmaceutical Co., Ltd. in Japan, which improves cachexia by increasing appetite, but its marketing application in the European Union was not approved, and it is still in Phase I clinical trials in China.

In the invention, the term "oligonucleotide" refers to a molecule composed of multiple linked nucleotides, each nucleotide comprising a sugar (e.g., ribose or deoxyribose) and an organic base attached thereto. Organic bases include pyrimidines (e.g., cytosine (C), thymine (T), or uracil (U)) or purines (e.g., adenine (A) or guanine (G)). Oligonucleotides are classified into DNA and RNA according to glycosyl groups. In the invention, an oligonucleotide may be unmodified or modified. Modification types include base modification, sugar modification, backbone modification, etc. In certain embodiments, the oligonucleotide of the invention comprises a phosphorothioate backbone modification, so that the oligonucleotide is resistant to nucleases and the stability of the oligonucleotide is improved. The preparation method of the oligonucleotide of the invention is not limited, and it can be synthesized *in vitro* or obtained by biotechnological or bioengineering methods. These methods are also known in the art.

In certain embodiments, the oligonucleotide of the invention is capable of restoring the expression of a gene suppressed in the cachexia state. The "seed" sequence of the oligonucleotide is: 5'→3': UCUGUGCC or TCTGTGCC; its reverse complementary sequence is: 5'→3': GGCACAGA; 0-8 random nucleotides may be present before the designed "seed" sequence, and 6-15 random nucleotides may be present after the designed "seed" sequence. Exemplarily, the oligonucleotide of the invention has a sequence as shown in SEQ ID NO. 1-42 below:

| Oligonucleotide Sequence | ID | Oligonucleotide Sequence | ID |
|---|---|---|---|
| ACUGCCUGUCUGUGCCUGCUGUAC | SEQ ID NO.1 | ACTGCCTGTCTGTGCCTGCTGTAC | SEQ ID NO.22 |
| ACUGCCUGUCUGUGCCUGCUGUA | SEQ ID NO.2 | ACTGCCTGTCTGTGCCTGCTGTA | SEQ ID NO.23 |
| CUGCCUGUCUGUGCCUGCUGUA | SEQ ID NO.3 | CTGCCTGTCTGTGCCTGCTGTA | SEQ ID NO.24 |
| UGCCUGUCUGUGCCUGCUGUAC | SEQ ID NO.4 | TGCCTGTCTGTGCCTGCTGTAC | SEQ ID NO.25 |
| ACUGCCUGUCUGUGCCUGCUGU | SEQ ID NO.5 | ACTGCCTGTCTGTGCCTGCTGT | SEQ ID NO.26 |
| CUGCCUGUCUGUGCCUGCAGUA | SEQ ID NO.6 | CTGCCTGTCTGTGCCTGCAGTA | SEQ ID NO.27 |
| CUGCCUGUCUGUGCCUGCUGUAC | SEQ ID NO.7 | CTGCCTGTCTGTGCCTGCTGTAC | SEQ ID NO.28 |
| CCUGUCUGUGCCUGCUGUACA | SEQ ID NO.8 | CCTGTCTGTGCCTGCTGTACA | SEQ ID NO.29 |
| ACUGCCUGUCUGUGCCUGCUGUGUC A | SEQ ID NO.9 | ACTGCCTGTCTGTGCCTGCTGTGTC A | SEQ ID NO.30 |
| ACUGCCUGUCUGUGCCUGCUGUACA | SEQ NO.10 | ACTGCCTGTCTGTGCCTGCTGTACA | SEQ ID NO.31 |
| CUGCCUGUCUGUGCCUGCUGUACA | SEQ NO.11 | CTGCCTGTCTGTGCCTGCTGTACA | SEQ ID NO.32 |
| UGCCUGUCUGUGCCUGCUGUACA | SEQ NO.12 | TGCCTGTCTGTGCCTGCTGTACA | SEQ ID NO.33 |
| GCCUGUCUGUGCCUGCUGUACA | SEQ NO.13 | GCCTGTCTGTGCCTGCTGTACA | SEQ ID NO.34 |
| UCCUGUCUGUGCCUGCUGUACA | SEQ ID NO.14 | TCCTGTCTGTGCCTGCTGTACA | SEQ ID NO.35 |
| ACUGCCUGUCUGUGCCUGCUGA | SEQ ID NO.15 | ACTGCCTGTCTGTGCCTGCTGA | SEQ ID NO.36 |
| ACUGCCUGUCUGUGCCUGGAGU | SEQ ID NO.16 | ACTGCCTGTCTGTGCCTGGAGT | SEQ ID NO.37 |
| ACUGCCUGUCUGUGCCUGCAGU | SEQ ID NO.17 | ACTGCCTGTCTGTGCCTGCAGT | SEQ ID NO.38 |
| ACUGCCUGUCUGUGCCUGCACU | SEQ NO.18 | ACTGCCTGTCTGTGCCTGCACT | SEQ ID NO.39 |
| ACUGCCUGUCUGUGCCUACUGU | SEQ ID NO.19 | ACTGCCTGTCTGTGCCTACTGT | SEQ ID NO.40 |
| ACUGCCUGUCUGUGCCUGAUGU | SEQ ID NO.20 | ACTGCCTGTCTGTGCCTGATGT | SEQ ID NO.41 |
| AAUGCCUGUCUGUGCCUGCUGU | SEQ ID NO.21 | AATGCCTGTCTGTGCCTGCTGT | SEQ ID NO.42 |

In the invention, the term "nucleic acid" refers to a linear fragment of single-stranded or double-stranded deoxyribonucleic acid (DNA) or ribonucleic acid (RNA), which can be generated from any source. The length of the nucleic acid of the invention is not limited, including long-chain nucleic acids or short-chain nucleic acids. A single strand of a long-chain nucleic acid generally consists of more than 200 nucleotides, i.e., the single-strand length is more than 200 nt. Short-chain nucleic acids are sometimes also referred to as oligonucleotides, which are usually single-stranded and generally consist of 5-200 nucleotides, i.e., the length is 5-200 nt.

In certain embodiments, the nucleic acid of the invention may comprise the oligonucleotide of the invention. For example, the nucleic acid of the invention may be in the form of a nucleic acid construct. The nucleic acid of the invention may comprise at least one oligonucleotide having a sequence shown herein, for example, two, three, more than three or more oligonucleotides. In the case of multiple oligonucleotides shown herein, the linkage manner between the oligonucleotides is not particularly limited, and for example, a suitable spacer sequence may be used to link with the oligonucleotides. Exemplarily, the nucleic acid comprises at least one oligonucleotide selected from SEQ ID NO. 1-42.

In certain embodiments, the nucleic acid of the invention is a nucleic acid molecule capable of generating the oligonucleotide of the invention, for example, the nucleic acid generates the oligonucleotide of the invention through transcription. In this case, the nucleic acid comprises a nucleic acid expressing a sequence shown in any one of SEQ ID NO. 1-21. In certain embodiments, the nucleic acid may be part of an expression vector. An "expression vector" refers to a recombinantly or synthetically produced nucleic acid construct carrying a series of specific nucleic acid elements capable of transcribing a specific gene in a host cell, such as a promoter element, an enhancer element, and a selection element. Generally, gene expression is placed under the control of certain regulatory elements, such as constitutive or inducible promoters. In the invention, the vector may be a plasmid, a phagemid, a virus (including but not limited to adenovirus, lentivirus), and other types of vectors derived from viral or bacterial sources. The gene sequence for expressing the oligonucleotide of the invention may be operably linked to one or more elements of the vector, such as a promoter element. "Operably linked" is used to describe the linkage between a regulatory element and a gene or its coding region. That is, gene expression is usually placed under the control of certain regulatory elements, and a gene or coding region is "operably linked" to a regulatory element means that the gene or coding region is controlled or affected by the regulatory element.

In the invention, the oligonucleotide can be used for prevention and treatment of cachexia in the form of "extracellular nucleic acid". The oligonucleotide is an isolated and pure nucleic acid molecule and can stably exist in an extracellular environment, including but not limited to blood, saliva, gastric juice, etc. This is not only effective for orthotopic tumors but also of great significance for metastatic tumors or distant tumors, because the oligonucleotide of the invention can reach the target (target nucleic acid) through the circulatory system, making this novel systemic treatment solution an alternative to radiotherapy and chemotherapy drugs, thereby avoiding the toxic and side effects caused by radiotherapy and chemotherapy to patients and benefiting patients in cachexia treatment.

The term "isolated" refers to a substance and/or entity that is (1) separated from at least some components with which it was originally associated (whether in nature and/or in an experimental setting), and/or (2) produced, prepared, and/or manufactured by humans. An isolated substance and/or entity may be separated from at least about 10%, about 20%, about 30%, about 40%, about 50%, about 60%, about 70%, about 80%, about 90%, about 95%, about 98%, about 99%, substantially 100%, or 100% of the other components with which it was originally associated. In some embodiments, the purity of the isolated reagent is greater than about 80%, about 85%, about 90%, about 91%, about 92%, about 93%, about 94%, about 95%, about 96%, about 97%, about 98%, about 99%, substantially 100%, or 100%. A substance is "pure" if it is substantially free of other components. In addition, an "isolated cell" refers to a cell not contained in a multicellular organism.

The invention also provides a method of reversing, preventing, improving, or treating cachexia or related disorders in a subject in need thereof, comprising the step of administering to the subject in need thereof a therapeutically effective amount of a nucleic acid, wherein the nucleic acid comprises or can generate an oligonucleotide having a sequence shown in any one of SEQ ID NO. 1-42. It can be understood that the method may be therapeutic or prophylactic.

In a preferred embodiment of the use and method according to the invention, the vector used for the nucleic acid capable of generating the oligonucleotide sequence includes a recombinant plasmid, an AAV viral vector, a lentiviral vector, or a retroviral vector. In a specific embodiment, the vector used for the nucleic acid capable of generating an oligonucleotide having a sequence shown in any one of SEQ ID NO. 1-21 is an animal cell expression vector.

It can be understood that the nucleic acid or oligonucleotide of the invention may further be contained in a suitable delivery vector, and such delivery vectors are known in the art, including but not limited to: extracellular vesicles, especially exosomes, microvesicles, etc.; nanoparticles such as chitosan, polylactic-co-glycolic acid (PLGA), dendrimers; typical liposomes, etc. In a preferred embodiment, the oligonucleotide of the invention is contained in an extracellular vesicle, such as an exosome.

In a preferred embodiment, the method of the invention comprises the step of administering to a subject an animal cell expression vector, wherein the vector is capable of generating an oligonucleotide having a sequence shown in any one of SEQ ID NO. 1-21.

In a preferred embodiment, the method comprises the step of administering to subject cells transformed with a vector generating the oligonucleotide sequence. In one aspect, introduction of the plasmid of the invention into cells can be performed by any suitable method for introducing a vector into cells (e.g., gene transfer) such as transfection and transduction, which is not particularly limited.

In a preferred embodiment, the method comprises the step of administering to a subject an extracellular vesicle containing the oligonucleotide sequence, especially an exosome (or exosome-like particle). At least part of the membrane of the exosome or extracellular vesicle may be directly derived from cells or prepared by artificial synthesis. The particle size of the exosome or extracellular vesicle is not particularly limited, and it may have a micron-scale or even nanoscale particle size.

In a most preferred embodiment, the purpose of reversing, preventing, improving, or treating cachexia or related disorders is achieved by administering to a subject in need thereof a therapeutically effective amount of a recombinant plasmid generating an oligonucleotide having a sequence shown in any one of SEQ ID NO. 1-21.

In the invention, the drug or composition containing the nucleic acid or oligonucleotide may further comprise a pharmaceutically acceptable adjuvant. The term "pharmaceutically acceptable" refers to a carrier, excipient, or stabilizer that is non-toxic to cells or mammals at the dosages and concentrations employed or has an acceptable toxicity level determined by those skilled in the art. A non-limiting example of a pharmaceutically acceptable adjuvant is an aqueous pH buffer solution. Pharmaceutically acceptable adjuvants may also include one or more of the following: antioxidants such as ascorbic acid; low molecular weight (less than about 10 residues) polypeptides; proteins such as serum albumin; gelatin; immunoglobulins; hydrophilic polymers such as polyvinylpyrrolidone; amino acids; carbohydrates such as glucose, mannose, or dextrin; chelating agents such as EDTA; sugar alcohols such as mannitol or sorbitol; salt-forming counterions such as sodium; and nonionic surfactants such as Tween and polyethylene glycol (PEG).

Without being bound by any theory, the oligonucleotide of the invention may have a nucleotide sequence shown in any one of SEQ ID NO. 1-42 or a sequence obtained by chemical modification thereof. In addition, variant sequences of the above sequences are also within the protection scope of the invention. The term "variant sequence" refers to an oligonucleotide sequence that is not completely identical to the oligonucleotide sequence of the invention but retains one or more functional characteristics of the oligonucleotide sequence of the invention. In some aspects, a variant sequence of an oligonucleotide sequence or a chemically modified sequence retains all functional characteristics of the oligonucleotide sequence, including but not limited to binding activity to a target gene, inhibitory activity on target expression or target cells, regulatory activity on target-regulated downstream signaling pathways, and/or efficacy of reversing, preventing, improving, or treating cachexia. Preferably, the modified oligonucleotide does not result in loss of the function of the oligonucleotide to inhibit nucleic acid factors and/or inhibit their expression. The regulatory activity on target-regulated downstream signaling pathways further includes regulating related proteins, especially regulating the expression of proteins such as A2AR, and ultimately significantly altering the glycolytic metabolic state unique to cachexia.

In the invention, the inhibitory activity of an oligonucleotide on target expression or target cells can be determined by methods known in the art, which is not particularly limited. For example, real-time fluorescent quantitative PCR, Northern Blotting, and other methods can be used to quantify nucleic acid factors. For another example, CCK8 can be used to detect the inhibition rate of oligonucleotides on target cells, and the oligonucleotides of the invention have a high inhibition rate on target cells. In addition, the oligonucleotides shown in SEQ ID NO. 1-42 of the invention have a significant inhibitory effect on the expression of target genes (nucleic acid factors) and/or the function of nucleic acid factors.

Those skilled in the art can understand that GC content optimization can be performed according to the sequences shown in SEQ ID NO. 1-42 of the invention to further improve the affinity of oligonucleotides to target sequences, thereby affecting their inhibition efficiency.

In the invention, chemical modification of oligonucleotides to improve their stability, activity, or half-life is known in the art. The oligonucleotide of the invention takes a nucleotide group (or nucleotide residue) as a basic structural unit, wherein the nucleotide group contains a phosphate group, a ribose group, and a base. Preferably, the oligonucleotide contains at least one modified nucleotide group. The modified nucleotide group does not result in loss of the function of the oligonucleotide to inhibit nucleic acid factors and/or inhibit their expression.

In the invention, the modified nucleotide group is a nucleotide group in which a phosphate group and/or a ribose group is modified. For example, modification of a phosphate group refers to any modification of the phosphate group, including but not limited to phosphorothioate, boranophosphate, etc., such as replacing oxygen in the phosphate group with sulfur and borane, respectively. Modification of the phosphate group can stabilize the nucleic acid structure and maintain high specificity and affinity of base pairing. Preferably, a phosphorothioated nucleotide group refers to a nucleotide group in which non-bridging oxygen atoms in all phosphodiester bonds are replaced with sulfur atoms.

Modification of a ribose group refers to any modification of the ribose group. For example, introduction of certain substituents such as methoxy or fluorine at the 2'-hydroxyl position of the ribose group makes ribonucleases less likely to cleave the nucleic acid, thereby increasing the stability of the nucleic acid and making the nucleic acid more resistant to nuclease hydrolysis. Modifications of the 2'-hydroxyl group in nucleotide pentose include 2'-fluoro modification, 2'-methoxy (2'-OME) modification, 2'-methoxyethyl (2'-MOE) modification, 2'-2,4-dinitrophenol (2'-DNP) modification, locked nucleic acid (LNA) modification, 2'-amino modification, 2'-deoxy modification, etc.

In the invention, a variant sequence of an oligonucleotide sequence may include an oligonucleotide obtained by substitution, deletion, and/or addition of one or more nucleotides to the nucleotide sequence shown in any one of SEQ ID NO. 1-42 and having the same function, and whether the variant sequence has the same function as the specified sequence in different cases can be determined based on any one specified sequence from the above sequences. In specific embodiments, substitution, deletion, and/or addition refer to substitution, deletion, and/or addition of one or more nucleotides selected from A, G, C, and T bases.

In certain embodiments, nucleotide insertions include 5' and/or 3' terminal insertions, as well as intra-sequence insertions of single or multiple residues (including at least 1, 2, 3 nucleotides).

In certain embodiments, nucleotide deletions refer to removal of one or more residues from an oligonucleotide sequence. These deletion variants are usually prepared by site-specific mutagenesis of nucleotides in DNA encoding the oligonucleotide to produce DNA encoding the variant, followed by expression of the DNA in recombinant cell culture. Alternatively, deletion variants can be conveniently prepared by *in vitro* synthesis.

In certain embodiments, nucleotide substitutions are those in which, for example, at least 1 to at least 3 nucleotides of the sequence have been removed and different nucleotides inserted at their positions. Although the sites for introducing sequence variations are predetermined, the mutations themselves need not be predetermined. For example, to optimize the performance of mutations at a given site, random mutagenesis can be performed at the target region, and the expressed substitution variants are screened for the optimal combination of desired activities. Techniques for introducing substitution mutations at predetermined sites in DNA having a known sequence are well known in the art.

In the invention, nucleotide substitutions are usually single residues, nucleotide insertions are usually about 1-10 residues, and nucleotide deletions are usually about 1-30 residues.

In the invention, a variant sequence of an oligonucleotide sequence may be a nucleotide sequence having 95%, such as 96%, 97%, 98%, or even 99% or more homology with the nucleotide sequence shown in any one of SEQ ID NO. 1-42 and having the same function. Similarly, whether a variant sequence with the above homology has the same function as the specified sequence can be determined based on any one specified sequence from the above sequences.

Sequence alignment can be performed to determine sequence homology (sometimes referred to as "identity"), which can be done by various methods known to those skilled in the art, such as using BLAST, BLAST-2, ALIGN, NEEDLE, or Megalign (DNASTAR) software. Those skilled in the art can determine appropriate parameters for alignment, including any algorithms needed to achieve optimal alignment over the full-length sequences being compared.

The dosage form of the drug or composition containing the nucleic acid or oligonucleotide of the invention is not particularly limited, and may be selected from an injection, an injectable powder, a tablet, an ointment, a capsule, a granule, an aerosol, a spray, or a powder for inhalation. In a preferred embodiment, the dosage form of the drug or composition containing the nucleic acid or oligonucleotide of the invention is an injection.

In the invention, the administration route of the drug or composition is not particularly limited, including but not limited to systemic administration by subcutaneous injection, intramuscular injection, intravenous administration, oral administration, inhalation administration, or sustained-release administration.

In a preferred embodiment, the drug or composition is an injection suitable for parenteral administration, especially intravenous administration. In a most preferred embodiment, the purpose of reversing, improving, or treating cachexia or related disorders is achieved by intravenously administering to a subject in need thereof a therapeutically effective amount of a recombinant plasmid vector containing an oligonucleotide.

In the invention, the term "prevention and treatment" refers to therapeutic treatment and prophylactic or preventive measures, the purpose of which is to prevent or slow down (reduce) undesirable physiological changes or disorders, such as the progression of cachexia. The degree of such alleviation or prevention is at least 5%, 10%, 20%, 40%, 50%, 60%, 80%, 90%, 95%, or 100% as measured according to any standard technique compared with an untreated control group under equivalent conditions. Beneficial or desired clinical results include, but are not limited to, the following results, whether detectable or undetectable: relief of symptoms, reduction in the severity of the disease, stable (i.e., not worsening) disease state, delayed or slowed disease progression, improvement or palliation of the disease state, and reduction (whether partial or total) of the disease. Those in need of treatment include those already suffering from cancer or cachexia-related diseases or those in need of prevention or improvement of cancer or cachexia-related diseases.

The term "subject" as used herein refers to any animal (e.g., a mammal), including but not limited to a human, a non-human primate, a rodent, and the like, to be subjected to a specific treatment. Generally, "subject" and "patient" are used interchangeably in the invention and both refer to a human subject.

The term "therapeutically effective amount" as used herein refers to a dose that is pharmaceutically effective, i.e., the amount of active drug (i.e., nucleic acid or oligonucleotide) is sufficient to significantly improve the condition without causing serious side effects. The dosage depends on many factors, such as the nature and severity of the disease to be prevented or treated, the gender, age, weight, personality, and individual response of the patient or animal, the administration route, the number of administrations, and the purpose of treatment, so the therapeutic dosage of the invention can vary widely.

Although therapeutic dosages can vary widely, the inventors have found that the administered nucleic acid or oligonucleotide can achieve an effective treatment for cachexia at a low dose. For an individual, e.g., an individual weighing 60 kg, the daily dosage of the nucleic acid or oligonucleotide is usually 4 µg per person per time to 60 µg per person per time, preferably 5-50 µg per person per time. Exemplary effective dosages include 5 µg per person per time, 6 µg per person per time, 7 µg per person per time, 8 µg per person per time, 9 µg per person per time, 10 µg per person per time, 11 µg per person per time, 12 µg per person per time, 13 µg per person per time, 14 µg per person per time, 15 µg per person per time, 16 µg per person per time, 17 µg per person per time, 18 µg per person per time, 19 µg per person per time, 20 µg per person per time, 21 µg per person per time, 22 µg per person per time, 23 µg per person per time, 24 µg per person per time, 25 µg per person per time, 26 µg per person per time, 27 µg per person per time, 28 µg per person per time, 29 µg per person per time, 30 µg per person per time, 31 µg per person per time, 32 µg per person per time, 33 µg per person per time, 34 µg per person per time, 35 µg per person per time, 36 µg per person per time, 37 µg per person per time, 38 µg per person per time, 39 µg per person per time, 40 µg per person per time, 41 µg per person per time, 42 µg per person per time, 43 µg per person per time, 44 µg per person per time, 45 µg per person per time, 46 µg per person per time, 47 µg per person per time, 48 µg per person per time, 49 µg per person per time, 50 µg per person per time. It can be administered as a single dose once a day, multiple times a day, or intermittently.

In certain embodiments, the daily dosage of the nucleic acid or oligonucleotide is 0.005 µg/kg to 1 µg/kg, preferably 0.006-1 µg/kg. Exemplary effective dosages include 0.006 µg/kg, 0.007 µg/kg, 0.008 µg/kg, 0.009 µg/kg, 0.01 µg/kg, 0.02 µg/kg, 0.03 µg/kg, 0.04 µg/kg, 0.05 µg/kg, 0.06 µg/kg, 0.07 µg/kg, 0.08 µg/kg, 0.09 µg/kg, 0.1 µg/kg, 0.2 µg/kg, 0.3 µg/kg, 0.4 µg/kg, 0.5 µg/kg, 0.6 µg/kg, 0.7 µg/kg, 0.8 µg/kg, 0.9 µg/kg, 1 µg/kg.

In the invention, administration of the drug can make the effective concentration of the oligonucleotide in the systemic circulation 1×10⁴-1×10⁹ copies/µL, preferably 2×10⁴-1×10⁸ copies/µL, more preferably 2×10⁴-1×10⁷ copies/µL, such as 2×10⁴, 3×10⁴, 4×10⁴, 5×10⁴, 6×10⁴, 7×10⁴, 8×10⁴, 9×10⁴, 1×10⁵, 2×10⁵, 3×10⁵, 4×10⁵, 5×10⁵, 6×10⁵, 7×10⁵, 8×10⁵, 9×10⁵, 1×10⁶, 2×10⁶, 3×10⁶, 4×10⁶, 5×10⁶, 6×10⁶, 7×10⁶, 8×10⁶, 9×10⁶, 1×10⁷ copies/µL.

In the invention, administration of the drug can make the effective concentration of the oligonucleotide in a target organ 1×10⁴-1×10⁹ copies/mg based on the weight of the corresponding target organ, preferably 2×10⁴-1×10⁸ copies/mg, more preferably 2×10⁴-1×10⁷ copies/mg, such as 2×10⁴, 3×10⁴, 4×10⁴, 5×10⁴, 6×10⁴, 7×10⁴, 8×10⁴, 9×10⁴, 1×10⁵ 2×10⁵, 3×10⁵, 4×10⁵, 5×10⁵, 6×10⁵, 7×10⁵, 8×10⁵, 9×10⁵, 1×10⁶ 2×10⁶, 3×10⁶, 4×10⁶, 5×10⁶, 6×10⁶, 7×10⁶, 8×10⁶, 9×10⁶, 1×10⁷ copies/mg.

In the invention, the nucleic acid or oligonucleotide can also be combined with other drugs to prepare a combined administration drug composition. The other drug may or may not be any therapeutic agent for cachexia, which is not particularly limited. It should be noted that the selected other drug does not react undesirably with the nucleic acid or oligonucleotide of the invention to cause mutual inhibitory activity. When administered in combination with other drugs, such as chemotherapeutic agents, immune checkpoint inhibitors, anti-tumor drugs, and immunomodulators, such as anti-PD-1 antibodies and anti-PD-L1 antibodies, the dosage of the other drug is determined according to the specific drug type, cachexia type, and development stage, etc. Each dosage can be 0.5 mg/kg-30 mg/kg, preferably 1-20 mg/kg; for example, for a person weighing 60 kg, each dosage is usually 1 mg-1500 mg, such as 50 mg-1200 mg, or 100 mg-800 mg, 150 mg-600 mg, or 200 mg-500 mg; exemplary dosages per administration include 50 mg, 100 mg, 120 mg, 160 mg, 180 mg, 200 mg, 240 mg, 260 mg, 300 mg, 320 mg, 360 mg, 400 mg, 500 mg, 600 mg, 800 mg, 1000 mg, 1200 mg, etc.; administered at intervals, the administration frequency is, for example, once every 3-7 days or once every 1-5 weeks, such as once every 3 days, once every 5 days, once every 1 week, once every 10 days, once every 2 weeks, once every 3 weeks, once every 4 weeks, once every 5 weeks, etc. The specific dosage and administration frequency should take into account factors such as administration route and patient health status, which can be determined by skilled physicians according to conventional skills.

In the invention, the tumor includes but is not limited to a solid tumor or a non-solid tumor. The solid tumor includes but is not limited to breast cancer, prostate cancer, melanoma, osteosarcoma, neuroblastoma, pancreatic cancer, lung cancer, rhabdomyosarcoma, Ewing's sarcoma, bladder cancer, colon cancer, liver cancer, ovarian cancer, cervical cancer, nasopharyngeal cancer, laryngeal cancer, gastric cancer, kidney cancer, head and neck tumor, esophageal cancer, testicular cancer, thyroid cancer, or brain cancer. Non-solid tumors include but are not limited to hematological tumors such as leukemia and lymphoma, and nervous system tumors.

### Example 1

The culture, transfection, and collection of cells are exemplarily shown below by taking tumor cells with a high incidence of cachexia such as lung cancer, pancreatic cancer, intestinal cancer, gastric cancer, and liver cancer as examples.

Cell Resuscitation: Quickly take out the required cell line from the liquid nitrogen tank, place the cryotube in a 37-40°C water bath, and gently shake until only a small amount of ice remains; transfer the cell suspension in the cryotube to a 15 mL centrifuge tube containing complete medium, centrifuge at 1200 rpm for 10 min, transfer to a biological safety cabinet, and aspirate the supernatant; resuspend the cells with an appropriate amount of complete medium, then transfer to a culture flask, and observe uniform cell dispersion under a microscope; then transfer the culture flask to a CO₂ incubator and culture at 37°C with 5% CO₂ until the cell confluence reaches 80-90% for cell passage.

Cell Passage: Observe the cells in the culture flask under a microscope; when the confluence reaches 80-90%, transfer to a biological safety cabinet, aspirate the supernatant, then add PBS to wash 2-3 times, aspirate the PBS, add trypsin to digest the cells, and complete digestion when the cell morphology shrinks to round under a microscope; add an appropriate amount of medium, blow down the cells with a pipette, and collect the cell suspension into a centrifuge tube; centrifuge the cell suspension at 1200 rpm for 10 min and aspirate the supernatant; add an appropriate amount of medium to resuspend the cells, aliquot the cells into culture flasks at a certain ratio, transfer the culture flasks to a cell incubator, and culture at 37°C with 5% CO₂ until the cell number and confluence meet the experimental requirements for subsequent experiments.

Cell Plating: When the cell confluence in the culture flask reaches 80-90%, transfer to a biological safety cabinet, aspirate the medium, then add PBS to wash 2-3 times, aspirate the PBS, add an appropriate amount of trypsin to digest until the cell morphology shrinks to round, and terminate digestion; add an appropriate amount of medium, gently blow the cells with a pipette to detach them, collect the cell suspension into a centrifuge tube, and centrifuge at 1200 rpm for 10 min; aspirate the supernatant and add an appropriate amount of medium to resuspend the cells. Pipette 20 µL of cell suspension into a 1.5 mL EP tube, add trypan blue to the EP tube at a ratio of 1:1, mix well, pipette 10 µL of the suspension into a hemocytometer, and count the cells. Calculate and pipette a certain amount of cell suspension into a 12-well plate according to the counting result, and transfer the 12-well plate to a cell incubator for overnight culture at 37°C with 5% CO₂.

Cell Transfection: Prepare two centrifuge tubes A and B. Add basal medium and Lipo3000 to tube A, invert to mix, and let stand for 5 min; add P3000 and plasmid to tube B (buffer solvent for the control group, oligonucleotide expression plasmid for the experimental group, i.e., a plasmid solution that can transcribe to generate the target oligonucleotide), invert to mix. After preparation (Lipo3000:plasmid:P3000 = 1 µL:0.5 µg:1 µL), mix the reagents in tubes A and B and let stand for 15 min. Add the above mixture to the 12-well plate, take fixed-point photos, and transfer to a cell incubator for culture at 37°C with 5% CO₂.

Cell Collection: Observe the cell growth status of each treatment group at 3 h, 6 h, 24 h, 48 h, and 72 h after transfection. At the sampling time points, aspirate the supernatant from the 12-well plate and add PBS to wash; add trypsin to the 12-well plate for digestion, add an appropriate amount of complete medium after digestion, blow the cells evenly, and collect the cell suspension of each group; centrifuge at 1200 rpm for 10 min, aspirate the supernatant, add an appropriate amount of PBS to resuspend and aliquot, and store the cell suspension in a -80°C refrigerator for subsequent experiments.

### Example 2

Oligonucleotide expression plasmids were designed using exemplary sequences, and it was verified *in vitro* that the expression plasmids can transcribe to generate exemplary oligonucleotides in a variety of human tumor cell lines.

Taking the sequences of SEQ ID NO. 5 and SEQ ID NO. 19 as examples, oligonucleotide expression plasmids were designed, and tumor cell lines with a high incidence of cachexia were used. In this example, SK-Hep-1, HepG2, and H292 cell lines were used to verify that the oligonucleotide expression plasmids can correctly transcribe to generate SEQ ID NO. 5 and SEQ ID NO. 19.

### Experimental Method:

The culture, transfection, and collection of SK-Hep-1, HepG2, and H292 cell lines were performed as described in Example 1. The collected cells were used for RNA extraction, followed by detection of intracellular expression of oligonucleotides.

qPCR Quantitative Detection: Prepare a qPCR reaction system according to the ratio of DEPC water 5.9 µL; Taq PCR Mix (2×) 10 µL; T probe 1 µL; F primer 0.5 µL; R primer 1.5 µL; Rox reference dye 0.1 µL; template (reverse transcription product) 1 µL; perform qPCR quantitative detection according to the procedure shown in Table 1.

**Table 1 qPCR Reaction Procedure**

| Temperature(°C) | Time | Cycle number |
|---|---|---|
| 95 | 2.5 min | 1 |
| 95 | 15s | 40 |
| 58 | 30s | |
| 72 | 30s | |

The results show that oligonucleotide expression plasmids designed with exemplary sequences can transcribe to generate exemplary oligonucleotides in a variety of human tumor cell lines. The concentrations of transcribed oligonucleotide sequences at 24 h are shown in Table 2 below.

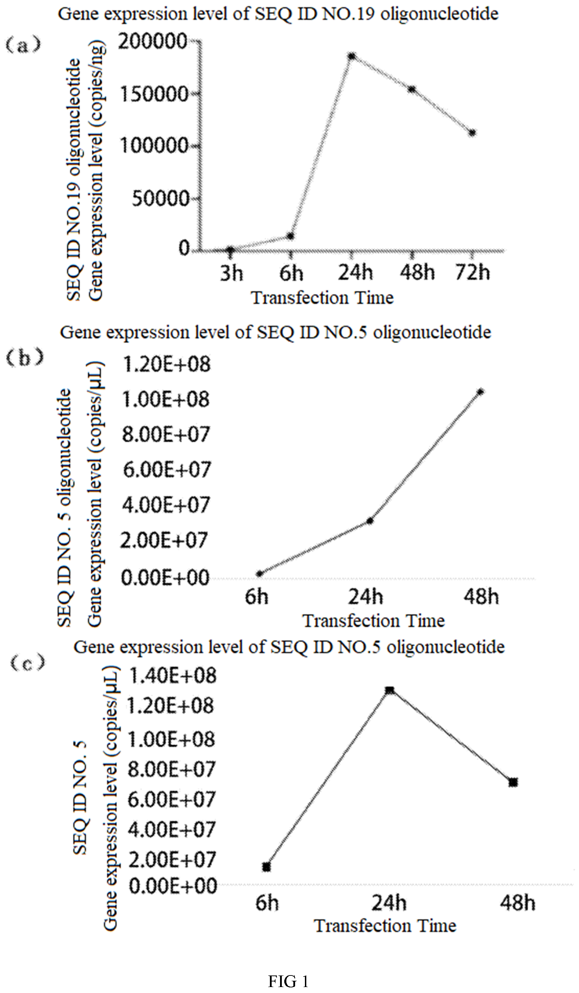

After entering the cells, the expression plasmid starts to express and generate oligonucleotide sequences, the concentration gradually accumulates and increases, and can be maintained for a long time, and still maintains a high concentration at 48 h. Different exemplary sequences show similar transcription accumulation and metabolic characteristics in different cells. As shown in (a), (b), and (c) of FIG 1, the expression plasmid designed based on SEQ ID NO. 19 can be efficiently transcribed in the SK-Hep-1 cell line, and the expression plasmid designed based on SEQ ID NO. 5 can be efficiently transcribed in the HepG2 and H292 cell lines, and the concentration gradually accumulates over time. The transcription results are highly consistent among different sequences and cell lines.

**Table 2 Oligonucleotide Expression Level at 24 h**

| **Exemplary Sequence Expression Plasmid** | **Cell Line** | **Oligonucleotide Expression Level at 24 h** |
|---|---|---|
| SEQ ID NO.19 Expression Plasmid | SK-Hep-1 | 1.86×10⁵copies/ng |
| SEQ ID NO.5 Expression Plasmid | HepG2 | 3.23×10⁷copies/µL |
| SEQ ID NO.5 Expression Plasmid | H292 | 1.33×10⁸copies/µL |

### Example 3

The expression of the A2AR gene is inhibited in the advanced tumor state, and nucleic acid or oligonucleotide sequences with exemplary characteristics can restore the expression of the A2AR gene, so that the mRNA abundance of the A2AR gene in human tumor cells returns to a high expression level.

Taking oligonucleotide expression plasmids designed with the sequences of SEQ ID NO. 5 and SEQ ID NO. 19 as examples, tumor cell lines with a high incidence of cachexia were used. In this example, MKN45, H292, and HepG2 cell lines were used to verify that oligonucleotides containing seed sequences can restore the expression of the A2AR gene.

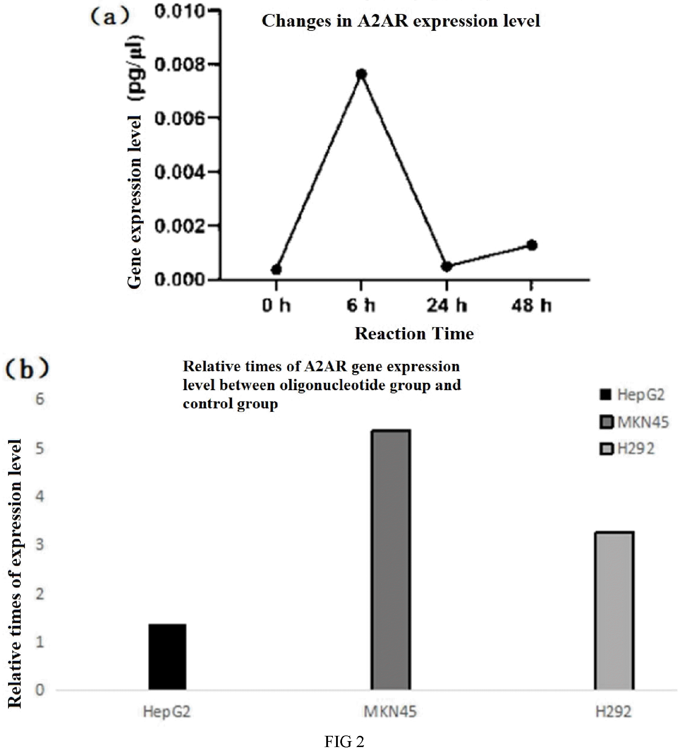

Taking the oligonucleotide expression plasmid designed with the sequence of SEQ ID NO. 19 as an example, the experimental results are shown in FIG 2(a). The mRNA of the A2AR gene can be restored and up-regulated within 48 h, with the highest abundance at 6 h.

Taking the oligonucleotide expression plasmid designed with the sequence of SEQ ID NO. 5 as an example, the experimental results are shown in FIG 2(b). The mRNA of the A2AR gene can be restored and up-regulated within 48 h in a variety of cell lines.

Experimental Method: The culture, transfection, and sample collection of MKN45, H292, and HepG2 cell lines were performed as described in Example 1. The collected cells were used for mRNA extraction, and the content of A2AR mRNA was detected by qPCR using the following primers.

Primers:
F: 5'-TGCTCGCTATCCCATTCG-3' (SEQ ID NO. 43);
R: 5'-GCGTGAGGACCAGGACAAA-3' (SEQ ID NO. 44).

### Example 4

After nucleic acid or oligonucleotide sequences with exemplary characteristics restore the expression of the A2AR gene, the abundance of A2AR protein in the cells is restored.

Taking oligonucleotide expression plasmids designed with the sequences of SEQ ID NO. 5, SEQ ID NO. 15, and SEQ ID NO. 19 as examples, tumor cell lines with a high incidence of cachexia were used. In this example, CW-2, CFPAC-1, BxPC-3, and SW620 cell lines were used to verify that after oligonucleotides containing seed sequences restore the mRNA expression of the A2AR gene, the abundance of A2AR protein also increases.

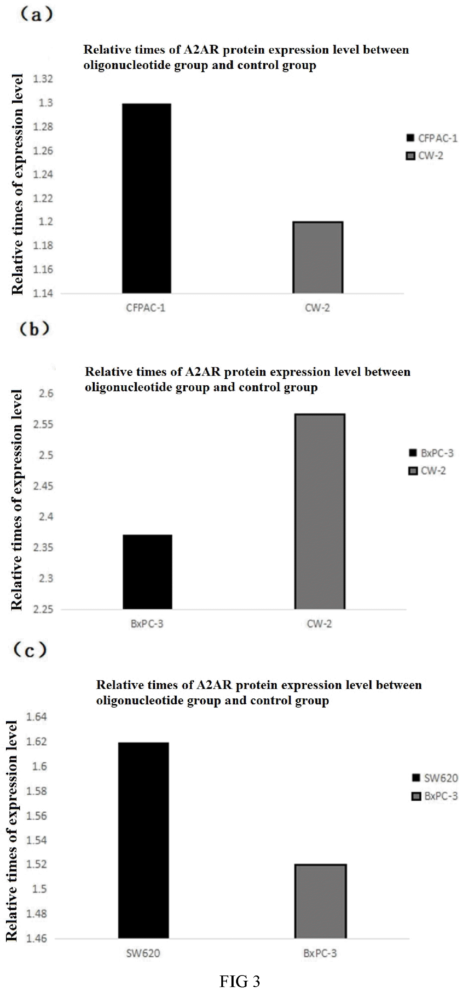

Taking the oligonucleotide expression plasmid designed with the sequence of SEQ ID NO. 5 as an example, the experimental results are shown in FIG 3(a). The expression level of A2AR protein in CFPAC-1 and CW-2 cells is significantly higher than that in the control group. Taking the oligonucleotide expression plasmid designed with the sequence of SEQ ID NO. 19 as an example, the experimental results are shown in FIG 3(b). The expression level of A2AR protein in CW-2 and BxPC-3 cells is significantly higher than that in the control group. Taking the oligonucleotide expression plasmid designed with the sequence of SEQ ID NO. 15 as an example, the experimental results are shown in FIG 3(c). The expression level of A2AR protein in BxPC-3 and SW620 cells is significantly higher than that in the control group.

It can be seen that after the exemplary oligonucleotide restores the expression of A2AR mRNA, the expression level of A2AR protein is also restored and up-regulated.

Experimental Method: The culture, transfection, and collection of CW-2, CFPAC-1, and BxPC-3 cell lines were performed as described in Example 1. The collected cells were used for protein extraction, and the abundance of A2AR protein was detected by ELISA.

### Example 5

This example studies that after chemical modification of nucleic acid or oligonucleotide sequences with exemplary characteristics, it is proved that the modified oligonucleotides can also restore the function of A2AR abundance.

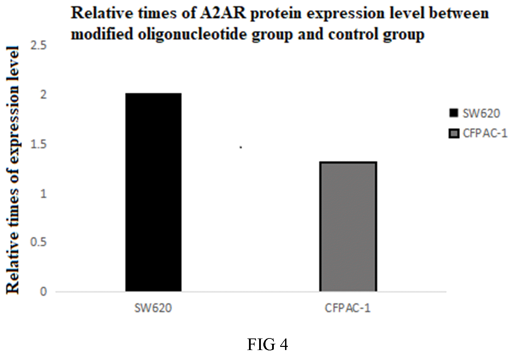

Taking the sequence of SEQ ID NO. 5 as an example, it was modified by phosphorothioate, fluorination, and methylation. Tumor cell lines with a high incidence of cachexia were used. In this example, SW620 and CFPAC-1 cell lines were used to verify that the chemically modified oligonucleotides with seed sequences can restore the abundance of A2AR protein. The results of A2AR protein expression are shown in FIG 4. It can be seen from FIG 4 that the expression level of A2AR protein in SW620 and CFPAC-1 cells is significantly higher than that in the control group. It can be seen that modification of the oligonucleotide sequence does not change its function of restoring A2AR abundance.

### Modification Mode:

mA*fC*mUmGmCfCmUfGfUmCmUmGmUfGmCfCmUmGmCmUmG*mU*mU
(m: methylation modification; *: phosphorothioate modification; f: fluorination modification).

Experimental Method: SW620 and CFPAC-1 cell lines were cultured as described in Example 1 and then transfected. For transfection, two centrifuge tubes A and B were prepared. Basal medium and Lipo3000 were added to tube A; basal medium and modified oligonucleotides were added to tube B. The reagents in tubes A and B were inverted to mix and let stand, then the mixture was added to the cells. The specific transfection conditions were in accordance with the instructions of the commercial transfection kit. After transfection, the cells were further cultured and collected as described in Example 1. The collected cells were used for protein extraction, and the abundance of A2AR protein was detected by ELISA.

### Example 6

Drugs for treating tumors such as chemotherapy and radiotherapy may cause or aggravate the process of cachexia. However, the oligonucleotides of the invention can treat cachexia while maintaining tumor stability and inhibiting tumor progression. This example further confirms that the series of sequences proposed by the invention have the potential to treat tumor cachexia by using tumor cell inhibition rate.

Examples 1-5 confirm that nucleic acids or oligonucleotides with the characteristics of the invention can accurately restore the expression of the A2AR gene. Taking the sequences of SEQ ID NO. 1, 2, 4, 5, 9, 13, 14, 15, and 19 as examples, tumor cell inhibition rates were studied in pancreatic cancer, colon cancer, lung cancer, and liver cancer cell lines with a high incidence of cachexia. The results show that all exemplary sequences of the invention show a certain inhibition rate, reflecting their potential to treat tumor cachexia. The detailed inhibition rates are shown in Table 3 below.

Experimental Method: The culture and transfection of H292, CFPAC-1, CW-2, SW620, BxPC-3, A549, H460, and HepG2 cell lines were performed as described in Example 1. After transfection, 10 µL of CCK8 reagent was added to the selected culture wells at an appropriate time point, then transferred to a cell incubator for culture at 37°C with 5% CO₂. After 1-2 h of culture, the OD450 value in the corresponding wells was detected with a microplate reader.

**Table 3 Inhibition Rate of Exemplary Sequences on Tumor Cells**

| Exemplary Sequence | Cell Line | Inhibition Rate |
|---|---|---|
| SEQ ID NO.1 | H292 cells | 80.2% |
| SEQ ID NO.2 | H292 cells | 54.2% |
| SEQ ID NO.4 | H292 cells | 57.2% |
| SEQ ID NO.5 | CFPAC-1 cells | 85.75% |
| SEQ ID NO.5 | CW-2 cells | 92.61% |
| SEQ ID NO.5 | SW620 cells | 94.43% |
| SEQ ID NO.5 | BxPC-3 cells | 73.6% |
| SEQ ID NO.5 | A549 cells | 52.7% |
| SEQ ID NO.9 | H292 cells | 47.0% |
| SEQ ID NO.13 | H292 cells | 48.2% |
| SEQ ID NO.14 | H460 cells | 62.1% |
| SEQ ID NO.14 | HepG2 cells | 78.3% |
| SEQ ID NO.15 | H292 cells | 30.8% |
| SEQ ID NO.19 | H292 cells | 57.13% |

Based on the above studies in Examples 1-6, it is confirmed that: the nucleic acid or oligonucleotide sequences with the characteristics of the invention proposed by the invention and oligonucleotides modified by nucleotides and having the same function can accurately restore the function of the A2AR gene in a variety of tumor cells, further regulate the expression of related pathway proteins, and finally achieve the purpose of treating cachexia. In addition, while cachexia is treated, it can significantly inhibit the growth of tumor cells. It is confirmed that the sequences of the invention have therapeutic potential for cachexia.

Typical symptoms of cachexia are weight loss and anorexia. The currently recognized etiologies are metabolic disorders, negative energy balance, etc. To further prove the therapeutic effect of the nucleic acid or oligonucleotide of the invention on tumor cachexia, we also simultaneously confirmed *in vivo* that A2AR can be released from inhibition in an *in vivo* model, then evaluated the therapeutic effect on anorexia by food intake, evaluated the therapeutic effect on weight loss by body weight, and carried out *in vivo* studies on the therapeutic effect of cachexia using an orthotopic tumor model.

The *in vivo* experimental results confirm that the use of oligonucleotides with exemplary characteristics can release the inhibition of A2AR and restore its expression in the orthotopic model, showing great therapeutic effect on tumor cachexia.

In the *in vivo* study, taking the sequence of SEQ ID NO. 5 as an example, tumor cell lines with a high incidence of cachexia were used. In Examples 7-10 of the invention, orthotopic tumor cachexia models were established using lung cancer H460, pancreatic cancer BxPC-3, and intestinal cancer SW620 cell lines, respectively, and treated with SEQ ID NO. 5, all achieving good therapeutic effects on cachexia. The detailed results are described in each example.

### Example 7

This example confirms that nucleic acids or oligonucleotides with the characteristics of the invention can also restore the expression of A2AR in an *in vivo* model, treat lung cancer cachexia, improve food and water intake, reduce the weight loss rate, maintain energy metabolic balance, and inhibit the progression of tumor cachexia.

Model Establishment: Thirty-six 6-week-old BALB/C nude mice were purchased, and after qualified quarantine and adaptive feeding, they were used for the establishment of a "nude mouse cachexia model". Four nude mice were subcutaneously inoculated with the cells cultured in Example 1 (H460 cell line) at an inoculation dose of 1×10⁷ cells·200 µL⁻¹·mouse⁻¹. After inoculation, the nude mice were caged by gender. When the subcutaneous tumor grew to an appropriate volume, the mice were euthanized, and the tumor was taken out under sterile conditions for orthotopic tumor inoculation.

After the tumor tissue was taken out and trimmed, the nude mice used for the establishment of the orthotopic tumor model were anesthetized, and the tumor tissue was inoculated under the left costal margin (lung tissue). After inoculation, the mice were transferred to a feeding cage, and samples were regularly collected to observe the model establishment status until the model was successfully established (i.e., weight loss >10% or tumor metastasis occurred).

*In vivo* Efficacy Study: After successful model establishment, the nude mice were injected with control reagent and oligonucleotide expression plasmid injection capable of expressing the sequence of SEQ ID NO. 5 via tail vein injection, respectively. The dosage was 0.6 mg/kg (the dosage calculated by oligonucleotide sequence was 2.7 µg/kg), the administration frequency was once every 1 day, and the administration was performed 7 times. During the administration, the body weight, clinical status, and diet indicators of the nude mice in each treatment group were observed and recorded. At the end of the experiment, the nude mice were dissected, the tumor number and volume indicators were observed and recorded, and the abundance of A2AR protein in tumor tissue was detected by ELISA.

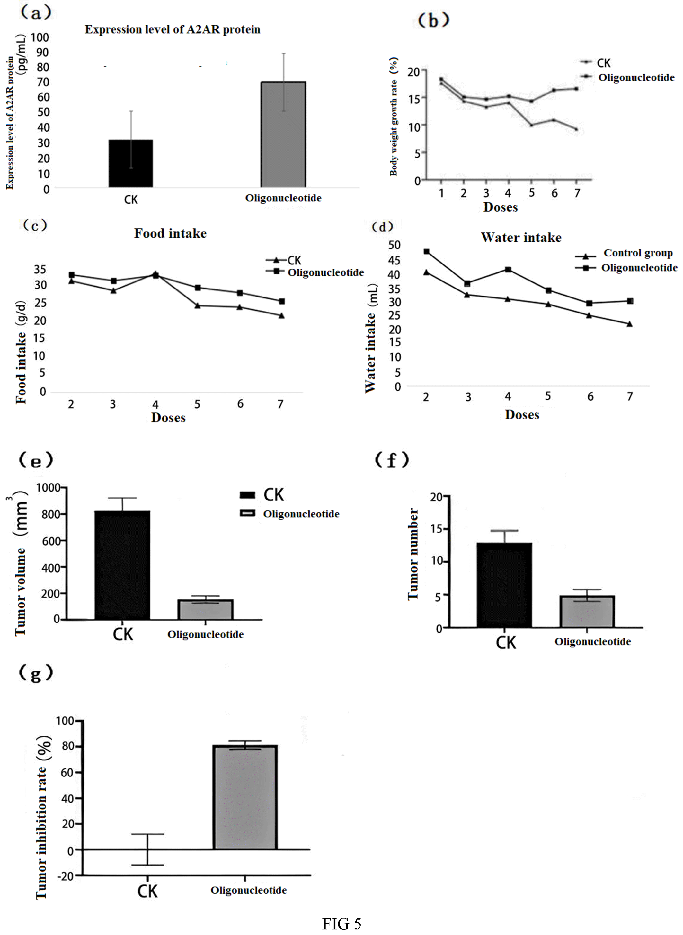

The experimental results are shown in FIG 5. FIG 5(a) shows that the abundance of A2AR in tumor tissue of the administration group at the end of the experiment is significantly higher than that of the control group. It confirms that administration of oligonucleotide sequences in the *in vivo* model can also release the inhibition of A2AR and restore its expression. FIG 5(b) shows that the body weight growth rate of nude mice in the oligonucleotide group is continuously stable during the administration period, significantly alleviating the core symptoms of cachexia, and the body weight at the end of the experiment is significantly higher than that of the control group. FIGs 5(c) and 5(d) show that from the second administration, the food intake (except the fourth administration) and water intake of nude mice in the oligonucleotide group are higher than those in the control group; it can be seen from (e), (f), and (g) of FIG 5 that at the end of the experiment, the tumor volume and tumor number of nude mice in the experimental group are reduced by 81.39% and 62.11% respectively compared with the control group, and tumor progression is inhibited; in addition, cage-side observation during the experiment shows that the mice have no diarrhea, poor condition, listlessness, arched back, cold body, or death during the whole medication period.

It shows that the exemplary sequences in the invention can restore the expression of A2AR protein, significantly improve the body weight, food intake, and water drinking behavior of nude mice with cachexia, effectively alleviate metabolic disorder symptoms, inhibit tumor development, and comprehensively improve the survival state of cachexia.

### Example 8

This example demonstrates that nucleic acids or oligonucleotides having the characteristics of the present invention can treat lung cancer cachexia *in vivo,* improve food and water intake, reduce body weight loss, maintain energy metabolic homeostasis, and inhibit the progression of tumor cachexia.

### Model Establishment: Same as Example 7.

*In vivo* Efficacy Evaluation: After successful model establishment, mice in each group were injected via tail vein with control vehicle and oligonucleotide expression plasmid injection capable of expressing SEQ ID NO. 5, respectively. The dosage was 0.2 mg/kg (equivalent to 0.9 µg/kg based on oligonucleotide sequence), administered once daily for 7 consecutive days. During administration, food intake and water intake of nude mice were observed and recorded; 16 mice were additionally monitored for body weight, tumor number and tumor volume at the experimental endpoint.

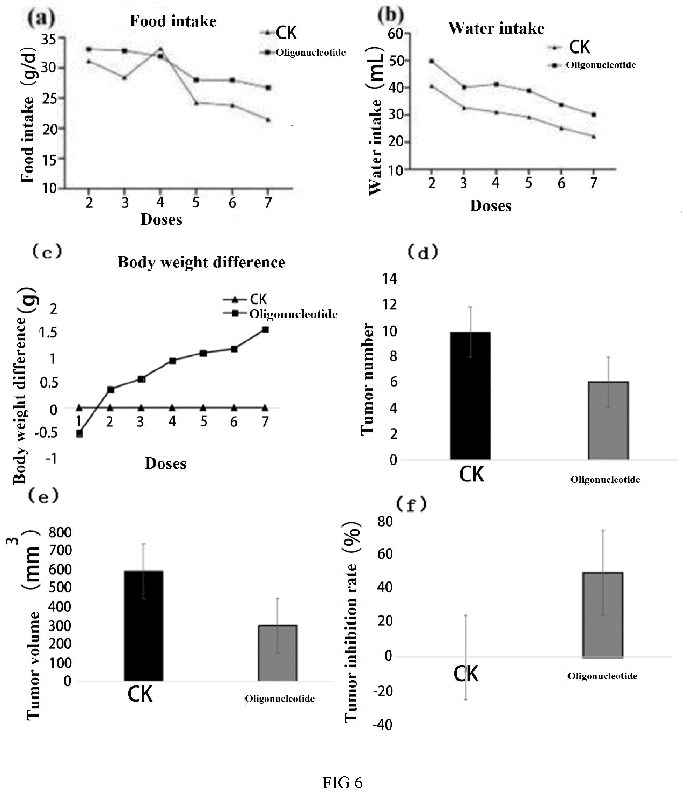

Experimental Results: As shown in FIG 6. (a)(b): From the second administration onward, food intake (except the fourth administration) and water intake of nude mice in the oligonucleotide group were consistently higher than those in the control group. (c): The body weight growth rate of nude mice in the oligonucleotide group remained stable during administration, significantly alleviating core cachexia symptoms, and body weight at the endpoint was markedly higher than that of the control group. (d)(e)(f): At the end of the experiment, tumor volume and tumor number in the experimental group were reduced by 49.67% and 39.13% respectively compared with the control group, indicating suppressed tumor progression.

The results indicate that the exemplary sequence in this example can significantly increase body weight, food intake and water consumption in cachectic nude mice, effectively alleviate metabolic disorders, inhibit tumor growth, and comprehensively improve the survival status of cachexia.

### Example 9

This example demonstrates that nucleic acids or oligonucleotides having the characteristics of the present invention can treat pancreatic cancer cachexia, improve food intake, reduce body weight loss rate, maintain energy metabolic homeostasis, increase survival rate during cachexia, and improve the survival status of cachexia.

Model Establishment: Twenty-four 6 week old BALB/C nude mice were purchased. BxPC 3 cell line was cultured as described in Example 1, and cells were directly inoculated orthotopically into the pancreatic tail of nude mice.

*In vivo* Efficacy Evaluation: After successful model establishment, nude mice were injected via tail vein with control vehicle and oligonucleotide expression plasmid injection capable of expressing SEQ ID NO. 5, respectively. The dosage was 0.4 mg/kg, administered once daily for 14 consecutive days. During administration, clinical status, food intake and other cachexia related indicators of nude mice in each treatment group were observed and recorded.

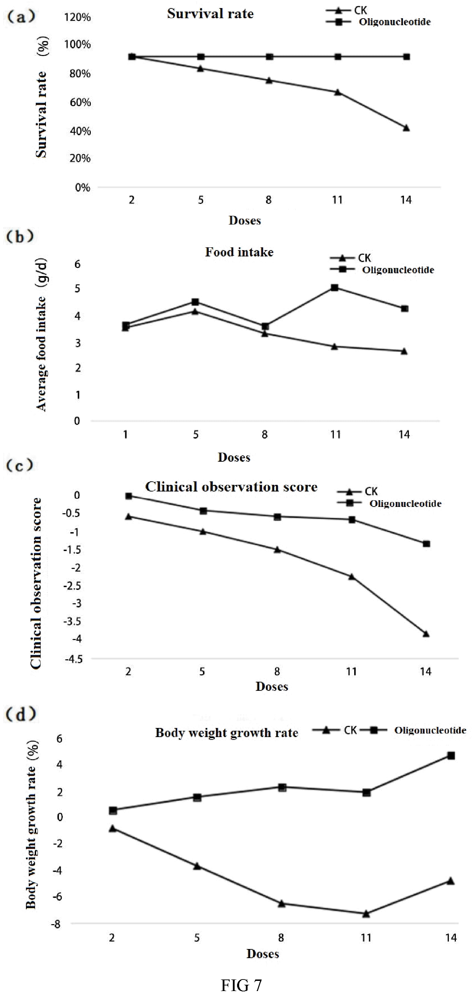

Experimental Results: As shown in FIG 7. (a): The survival rate of nude mice in the oligonucleotide group was superior to that in the control group throughout the experiment, proving that treatment with the exemplary oligonucleotide reduces the lethal risk of cachexia. (b): Food intake in the oligonucleotide group increased continuously during administration, with a significant difference widening after the 8th administration, indicating progressive restoration of metabolic homeostasis. (c): Clinical observation scores of nude mice in the oligonucleotide group were significantly better than those in the control group during administration. (d): The body weight growth rate of nude mice in the oligonucleotide group maintained steady growth, and the body weight loss rate was markedly better than that of the control group.

The results indicate that nucleic acids or oligonucleotides with the characteristics of the exemplary sequence in this example can treat pancreatic cancer cachexia, sustainably increase food intake, stabilize body weight growth rate, improve clinical status, and ultimately significantly improve the survival rate of cachexia models. It confirms that the treatment of cachexia by the exemplary sequence is centered on metabolic regulation, achieving dual improvements in quality of life and survival duration.

### Example 10

This example demonstrates that nucleic acids or oligonucleotides having the characteristics of the present invention can treat intestinal cancer cachexia, reduce body weight loss rate, maintain energy metabolic homeostasis, and also inhibit the progression of colorectal cancer.

Model Establishment: SW620 cell line was used. Nude mice were first inoculated subcutaneously with tumor cells as described in Example 7, then tumor tissue fragments were implanted orthotopically onto the serosal surface of the mesenteric side at 1 cm from the distal end of the cecum.

*In vivo* Efficacy Evaluation: After successful model establishment, nude mice were injected via tail vein with control vehicle and oligonucleotide expression plasmid injection capable of expressing SEQ ID NO. 5, respectively. The dosage was 0.1 mg/kg, administered once daily for 14 consecutive days. Body weight of nude mice in each treatment group was recorded during administration; mice were dissected on the day after the last administration, and orthotopic tumor size was measured.

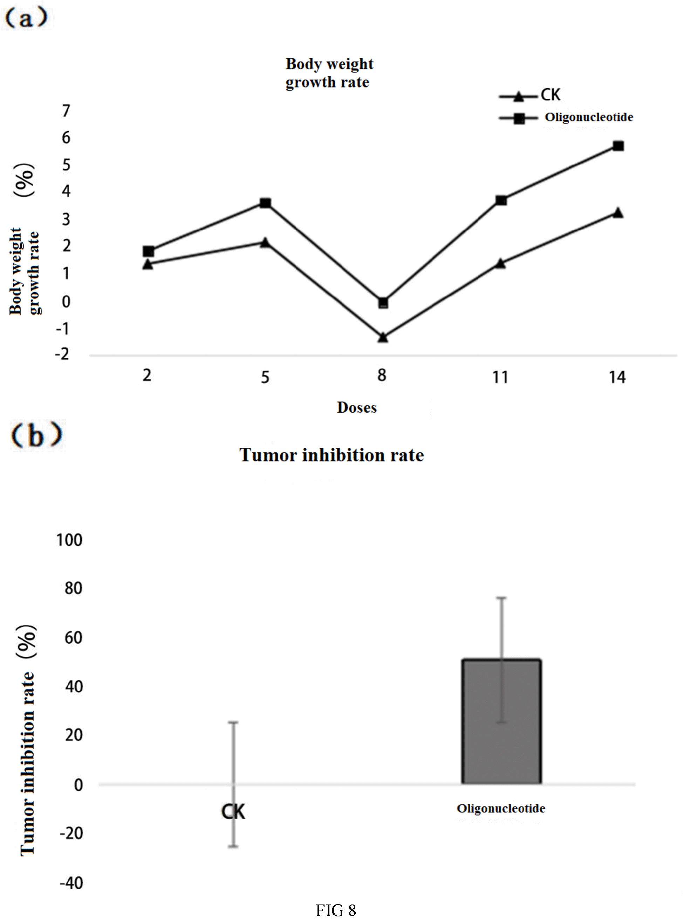

Experimental Results: As shown in FIG 8. (a): Body weight of nude mice in the oligonucleotide group kept increasing, with a growth rate consistently higher than that of the control group. (b): At the experimental endpoint, tumor volume in the administration group was reduced by 50.69% compared with the control group, indicating inhibited tumor progression.

The results indicate that the exemplary sequence in this example achieves a dual breakthrough in alleviating cachexia symptoms and inhibiting tumors in a highly metastatic colon cancer model.

General Conclusion of Examples 1-10: the above studies in Examples 1-10 confirm that the present invention creatively identifies a seed sequence capable of restoring A2AR gene expression. Vectors designed for stable transcription of such nucleic acids or oligonucleotides have been verified *in vitro* and *in vivo* to accurately restore A2AR gene expression, regulate the expression of downstream pathway proteins, increase appetite, maintain or even increase body weight, and ultimately reverse the pathological process of cachexia. In animal models, the core symptoms of cachexia are significantly improved (increased food intake, suppressed body weight loss, prolonged survival), providing a novel strategy for cachexia treatment.

This specification provides specific sequences of nucleic acids or oligonucleotides. In accordance with relevant regulations, the present application also provides a sequence listing in computer readable form. It should be noted that the sequences in the computer readable sequence listing are for reference only. In case of any inconsistency between the sequences in this specification and those in the computer readable sequence listing, the sequences in this specification shall prevail.

Finally, it should be noted that the above examples are only used to illustrate the technical solutions of the present invention rather than limit them. Although the present invention has been described in detail with reference to the foregoing examples, those of ordinary skill in the art should understand that: they can still modify the technical solutions described in the foregoing examples, or make equivalent replacements to some of the technical features thereof. However, these modifications or replacements do not cause the essence of the corresponding technical solutions to depart from the spirit and scope of the technical solutions of the examples of the present invention.

## Claims

1. An oligonucleotide, **characterized in that**, the oligonucleotide is an oligonucleotide of the following (1) and/or (2):
(1) an oligonucleotide having a sequence as set forth in any one of SEQ ID NO. 1-42;
(2) an oligonucleotide obtained by nucleotide modification of the oligonucleotide of (1) above with a same function.

2. The oligonucleotide according to claim 1, **characterized in that**, the oligonucleotide has a sequence as set forth in any one of SEQ ID NO.1, 2, 4, 5, 9, 13, 14, 15, 19, 22, 23, 25, 26, 30, 34, 35, 36 or 40.

3. The oligonucleotide according to any one of claims 1-2, **characterized in that**, the nucleotide modification comprises modification of a phosphate group, a ribose group and/or a conjugated linker;
wherein the modification of the phosphate group refers to modification of oxygen in the phosphate group, comprising phosphorothioate and boranophosphate, so as to stabilize a nucleic acid structure;
the modification of the ribose group refers to modification of a 2' hydroxyl group in the ribose group, comprising introduction of a methoxy group or a fluorine group at the 2' hydroxyl position of the ribose group, so that ribonucleases are less likely to cleave the nucleic acid and increase nucleic acid stability;
the modification of the conjugated linker comprises small molecule ligands, antibodies, peptides or carbon chains.

4. An expression vector, **characterized in that**, the expression vector comprises the oligonucleotide according to any one of claims 1-3, or is capable of producing the oligonucleotide according to claim 1 in a host.

5. The expression vector according to claim 4, **characterized in that**, the vector comprises a recombinant plasmid, an AAV viral vector, a lentiviral vector or a retroviral vector.

6. Use of the oligonucleotide according to any one of claims 1-3 or the expression vector according to any one of claims 4-5 in a manufacture of a medicine for preventing and treating cachexia.

7. The use according to claim 6, **characterized in that**, the preventing and treating comprises: preventing, alleviating, reversing, improving or curing cachexia or symptoms thereof.

8. The use according to claim 7, **characterized in that**, the cachexia comprises one or more of the pre cachexia stage, cachexia stage and refractory cachexia stage caused by tumor progression.

9. The use according to claim 8, **characterized in that**, the tumor comprises a solid tumor or a non-solid tumor;
the solid tumor comprises: gastric cancer, pancreatic cancer, lung cancer, intestinal cancer, prostate cancer, esophageal cancer, liver cancer, breast cancer, melanoma, osteosarcoma, neuroblastoma, rhabdomyosarcoma, Ewing's sarcoma, bladder cancer, ovarian cancer, cervical cancer, nasopharyngeal cancer, laryngeal cancer, kidney cancer, head and neck tumor, testicular cancer, thyroid cancer or brain cancer;
the non-solid tumor comprises: leukemia or lymphoma.

10. The use according to claim 9, **characterized in that**, the tumor comprises gastric cancer, lung cancer, intestinal cancer, pancreatic cancer or liver cancer.

11. The use according to claim 6, **characterized in that**, symptoms of the cachexia include muscle wasting, body weight loss, listlessness, insomnia, systemic failure, metabolic abnormalities, decreased food intake and/or decreased activity.

12. A pharmaceutical composition, **characterized in that**, the pharmaceutical composition comprises the oligonucleotide according to any one of claims 1-3 or the expression vector according to any one of claims 4-5.

13. The pharmaceutical composition according to claim 12, **characterized in that**, the pharmaceutical composition further comprises a pharmaceutically acceptable carrier.

14. The pharmaceutical composition according to claim 13, **characterized in that**, a dosage form of the medicine comprises injection, injectable powder, tablet, ointment, capsule, granule, aerosol, spray or powder for inhalation.

15. A method of administration of a nucleic acid containing medicine, **characterized in that**, the nucleic acid comprises or is capable of producing an oligonucleotide of the following (1) and/or (2):
(1) an oligonucleotide having a sequence as set forth in any one of SEQ ID NO. 1-42;
(2) an oligonucleotide obtained by nucleotide modification of the oligonucleotide of (1) above with a same function;
the method comprises administering the medicine via systemic administration.

16. The method of administration of a nucleic acid containing medicine according to claim 15, **characterized in that**, the method comprises systemic administration via subcutaneous injection, intramuscular injection, intravenous administration, oral administration, inhalation administration or sustained release administration.

17. The method of administration according to claim 15, **characterized in that**, the administration of the medicine is capable of delivering the oligonucleotide into body at a dose of 4 µg-60 µg per person per time based on a body weight of 60 kg.

18. The method of administration according to claim 17, **characterized in that**, the administration of the medicine is capable of delivering the oligonucleotide into the body at a dose of 5 µg-50 µg per person per time based on a body weight of 60 kg.

19. The method of administration according to claim 15, **characterized in that**, the administration of the medicine enables an effective concentration of the oligonucleotide in a systemic circulation of 1×10⁴-1×10⁹ copies/µL, or enables an effective concentration of the oligonucleotide in a target organ of 1×10⁴-1×10⁹ copies/mg.

20. The method of administration according to claim 19, **characterized in that**, administration of the medicine enables an effective concentration of the oligonucleotide in the systemic circulation of 2×10⁴-1×10⁸ copies/µL, or enables an effective concentration of the oligonucleotide in a target organ of 2×10⁴-1×10⁸ copies/mg.

21. A method for treating and/or preventing cachexia, **characterized in that**, the oligonucleotide according to any one of claims 1-3, the expression vector according to any one of claims 4-5, or the pharmaceutical composition according to any one of claims 12-14 is administered to a patient, or a patient is treated according to the method of administration according to any one of claims 15-20.

## Amended claims

### Amended claims under Art. 19.1 PCT

1. An oligonucleotide, **characterized in that**, the oligonucleotide is an oligonucleotide of the following (1) and/or (2):
(1) an oligonucleotide having a sequence as set forth in any one of SEQ ID NO. 1, 2, 6, 7, 9, 10, 12-23, 27, 28, 30, 31, 33-42;
(2) an oligonucleotide obtained by nucleotide modification of the oligonucleotide of (1) above with a same function.

2. The oligonucleotide according to claim 1, **characterized in that**, the oligonucleotide has a sequence as set forth in any one of SEQ ID NO. 1, 2, 9, 13, 14, 15, 19, 22, 23, 30, 34, 35, 36 or 40.

3. The oligonucleotide according to any one of claims 1-2, **characterized in that**, the nucleotide modification comprises modification of a phosphate group, a ribose group and/or a conjugated linker;
wherein the modification of the phosphate group refers to modification of oxygen in the phosphate group, comprising phosphorothioate and boranophosphate, so as to stabilize a nucleic acid structure;
the modification of the ribose group refers to modification of a 2' hydroxyl group in the ribose group, comprising introduction of a methoxy group or a fluorine group at the 2' hydroxyl position of the ribose group, so that ribonucleases are less likely to cleave the nucleic acid and increase nucleic acid stability;
the modification of the conjugated linker comprises small molecule ligands, antibodies, peptides or carbon chains.

4. An expression vector, **characterized in that**, the expression vector comprises the oligonucleotide according to any one of claims 1-3, or is capable of producing the oligonucleotide according to claim 1 in a host.

5. The expression vector according to claim 4, **characterized in that**, the vector comprises a recombinant plasmid, an AAV viral vector, a lentiviral vector or a retroviral vector.

6. Use of the oligonucleotide according to any one of claims 1-3 or the expression vector according to any one of claims 4-5 in a manufacture of a medicine for preventing and treating cachexia, **characterized in that** the oligonucleotide is an oligonucleotide of the following (1) and/or (2):
(1) an oligonucleotide having a sequence as set forth in any one of SEQ ID NO. 1-42;
(2) an oligonucleotide obtained by nucleotide modification of the oligonucleotide of (1) above with a same function.

7. The use according to claim 6, **characterized in that**, the preventing and treating comprises: preventing, alleviating, reversing, improving or curing cachexia or symptoms thereof.

8. The use according to claim 7, **characterized in that**, the cachexia comprises one or more of the pre cachexia stage, cachexia stage and refractory cachexia stage caused by tumor progression.

9. The use according to claim 8, **characterized in that**, the tumor comprises a solid tumor or a non-solid tumor;
the solid tumor comprises: gastric cancer, pancreatic cancer, lung cancer, intestinal cancer, prostate cancer, esophageal cancer, liver cancer, breast cancer, melanoma, osteosarcoma, neuroblastoma, rhabdomyosarcoma, Ewing's sarcoma, bladder cancer, ovarian cancer, cervical cancer, nasopharyngeal cancer, laryngeal cancer, kidney cancer, head and neck tumor, testicular cancer, thyroid cancer or brain cancer;
the non-solid tumor comprises: leukemia or lymphoma.

10. The use according to claim 9, **characterized in that**, the tumor comprises gastric cancer, lung cancer, intestinal cancer, pancreatic cancer or liver cancer.

11. The use according to claim 6, **characterized in that**, symptoms of the cachexia include muscle wasting, body weight loss, listlessness, insomnia, systemic failure, metabolic abnormalities, decreased food intake and/or decreased activity.

12. A pharmaceutical composition, **characterized in that**, the pharmaceutical composition comprises the oligonucleotide according to any one of claims 1-3 or the expression vector according to any one of claims 4-5.

13. The pharmaceutical composition according to claim 12, **characterized in that**, the pharmaceutical composition further comprises a pharmaceutically acceptable carrier.

14. The pharmaceutical composition according to claim 13, **characterized in that**, a dosage form of the medicine comprises injection, injectable powder, tablet, ointment, capsule, granule, aerosol, spray or powder for inhalation.

15. A method of administration of a nucleic acid containing medicine, **characterized in that**, the nucleic acid comprises or is capable of producing an oligonucleotide of the following (1) and/or (2):
(1) an oligonucleotide having a sequence as set forth in any one of SEQ ID NO. 1-42;
(2) an oligonucleotide obtained by nucleotide modification of the oligonucleotide of (1) above with a same function;
the method comprises administering the medicine via systemic administration.

16. The method of administration of a nucleic acid containing medicine according to claim 15, **characterized in that**, the method comprises systemic administration via subcutaneous injection, intramuscular injection, intravenous administration, oral administration, inhalation administration or sustained release administration.

17. The method of administration according to claim 15, **characterized in that**, the administration of the medicine is capable of delivering the oligonucleotide into body at a dose of 4 µg-60 µg per person per time based on a body weight of 60 kg.

18. The method of administration according to claim 17, **characterized in that**, the administration of the medicine is capable of delivering the oligonucleotide into the body at a dose of 5 µg-50 µg per person per time based on a body weight of 60 kg.

19. The method of administration according to claim 15, **characterized in that**, the administration of the medicine enables an effective concentration of the oligonucleotide in a systemic circulation of 1×10⁴-1×10⁹ copies/µL, or enables an effective concentration of the oligonucleotide in a target organ of 1×10⁴-1×10⁹ copies/mg.

20. The method of administration according to claim 19, **characterized in that**, administration of the medicine enables an effective concentration of the oligonucleotide in the systemic circulation of 10⁴-1×10⁸ copies/µL, or enables an effective concentration of the oligonucleotide in a target organ of 2×10⁴-1×10⁸ copies/mg.

21. A method for treating and/or preventing cachexia, **characterized in that**, the oligonucleotide according to any one of claims 1-3, the expression vector according to any one of claims 4-5, or the pharmaceutical composition according to any one of claims 12-14 is administered to a patient, or a patient is treated according to the method of administration according to any one of claims 15-20.
